# EUROPEAN PATENT SPECIFICATION

(11) **EP 0 235 090 B1**
(45) Date of publication and mention of the grant of the patent: **15.09.1993**
(21) Application number: 87810103.9
(22) Date of filing: 23.02.1987
(51) Int. Cl.: A61K 31/57

(54) **Composition useful in the treatment of estrogen deficiencies**
In der Behandlung von Östrogeninsuffizienz zu verwendende Zusammensetzung
Composé apte au traitement des déficiences en oestrogènes

(30) Priority: 27.02.1986 US 834263
(43) Date of publication of application: 02.09.1987
(73) Proprietor: WARNER-LAMBERT COMPANY, Morris Plains New Jersey 07950 (US)
(72) Inventor: Boissoneault, Roger, Long Valley, N.J. 07853 (US)
(74) Representative: Jones, Michael Raymond

(56) References cited:
- DICTIONNAIRE VIDAL, 1978, O.V.P., Paris (FR); "Primodos", p. 1639#
- DICTIONNAIRE VIDAL, 1970, O.V.P., Paris (FR); "Milli-anovlar", p. 912#
- DICTIONNAIRE VIDAL, 1973, O.V.P., Paris (FR); "Gynovlane", p. 746#

## Description

The treatment of menopausal symptoms such as osteoporosis and other ailments associated with oestrogen deficiency is old. Typically, the known formulations for such treatment have contained natural oestrogen or other oestrogenic component(s) as the only hormonal ingredient. The replacement of oestrogen with these types of formulations containing oestrogen only, has led to evidence of adenocarcinoma of the endometrium.

The use of natural progesterone or other progestogenic agents along with oestrogenic substances has been found to reduce various undesirable side effects associated with the use of oestrogen agents alone.

Combinations of oestrogenic and progestogenic agents have been used in the past to treat abnormalities of the menstrual cycle such as dysmennorrhia.

According to the present invention there is provided the use of a composition comprising 0.005 to 0.050 mg of ethinyl oestradiol and 0.1 to 1.0 mg of norethindrone acetate for use in the manufacture of a medicament for the treatment of osteoporosis by the daily administration of the composition for 20 to 30 days of a 30 to 35 day cycle.

According to the present invention there is also provided a package for use in the treatment of osteoporosis by daily administration of a medicament for 20 to 30 days of a cycle lasting 30 to 35 days, which package contains a medicament which includes as individual daily doses a composition comprising 0.005 to 0.050 mg of ethinyl oestradiol and 0.1 to 1.0 mg of norethindrone acetate, the medicament being arranged in the form of a dosage unit containing a sufficient quantity of daily doses for daily administration for 20 to 30 days of a cycle lasting 30 to 35 days.

It has been found that a fixed combination of oestrogenic and progestogenic agents gives relief from the menopausal symptom osteoporosis with minimal side effects. In one preferred embodiment, a composition containing a fixed dosage of ethinyl oestradiol, i.e., 0.005-0.05 mg, along with a fixed dosage of norethindrone acetate, i.e., 0.1-1.0 mg, for use in the manufacture of a medicament to be administered as a daily dosis in a 28-day sequence, gives acceptable oestrogen levels in patients.

Thus, the invention is concerned with the use of a formulation containing a fixed oestrogen/progestin ratio for the manufacture of a medicament to be administered to female individuals with resultant relief from osteoporosis.

The invention has several advantages over the known prior art. Principal among the advantages are:
the compositions contain fixed, i.e., constant or unitary, quantities of both the oestrogenic and progestogenic agents; this simplifies manufacturing, storage and packaging;
the use of a continuously dosed product minimizes patient compliance problems;
the administration of a single combination product containing fixed quantities of hormonal agents is psychologically beneficial.

The compositions generally contain 0.001 to 0.1 parts by weight, preferably 0.005 to 0.05 parts of the oestrogenic ingredient and 0.1 to 2.0 parts by weight, and preferably 0.1 to 1.0 parts by weight of the progestogenic ingredient.

Generally, the ratios by weight of oestrogenic to progestogenic components in the inventive compositions will be from 0.005:1 to 1:1, preferably 0.005:1 to 0.02:1.

While milligrams are the preferred units of measurement, any scale can be used so long as the ratio of the active hormonal ingredients remains fixed and is appropriate to the weight ratios set out above.

The oestrogenic ingredient of the compositions according to the present invention can be any suitable synthetic oestrogen or functional equivalent thereof. Ethinyl oestradiol is the preferred oestrogenic substance and must be present in the composition, but other useful substances include conjugated oestrogens, oestrone sulphate, beta oestradiol, quinestrol, and the like. Mixtures are operable.

The progestogenic ingredient is generally a synthetic progestogen: however, natural progestins may be used. Useful progestogenic substances include medroxy-progesterone acetate and the like. Norethindrone acetate is preferred and must be present for the compositions of the invention. Mixtures are operable.

While it is preferred that the synthetic oestrogen and progestin be the only pharmaceutically active ingredients in the compositions, the use of other drugs and/or otherwise beneficial substances in the instant compositions is contemplated.

The use of conventional pharmaceutical carriers is contemplated. Other excipients such as perfumes, colourants, stabilizers fillers, and the like can be used as well.

The compositions of the invention can be administrated via a variety of routes. Any method or combination of methods by which a continuous dosage form can be administered is operable. Oral dosage forms are preferred.

When oral dosage forms are employed, it is generally preferred that they be solid or semisolid. However, liquid compositions are contemplated.

The compositions of the invention may be packaged in a solid dosage form, in a pill case or compact for sequential administration. Thus, a package similar to that sometimes used for dispensing contraceptive pills, tablets, and the like can be employed. Thus, the individual who is to ingest the subject composition merely takes the pills, tablets, and/or capsule in a daily regimen in the sequence in which they are presented in the package.

In general, any dosage form and packaging concept can be used in combination. The composition is preferably administered at least once daily for a period of 20 to 30 days, preferably, approximately 28 days, during a total cycle of 30 to 35 days. One highly preferred regimen calls for continuous administration of the composition to a human female for approximately 28 days of an approximately 30-day cycle.

The compositions of the invention are useful for treating osteoporosis.
The invention is illustrated by the following example(s).

### Example 1

1.00g ethinyl oestradiol United States Pharmacoepia (U.S.P.), (0.5% dilution (=0.005 g dry substance), 5% excess) was combined with 0.5g norethindrone acetate USP. 8.66g Hydrous Fast Flo lactose U.S.P. and 7.00g corn starch N.F. (National Formulary) in a suitable liquids/solids PK blender equipped with intensifier bar. The ingredients were blended for five minutes. All mixing was done using the intensifier bar unless specified otherwise.

17.50g microcrystalline cellulose NF Powder was added to the resultant blend and mixed for 5 minutes. 34.64g Hydrous Fast Flo lactose U.S.P. was added and all ingredients were blended for 5 minutes.

Thereafter, 0.70g calcium stearate NF powder was added and blended with the intensifier bar for 1 minute and without it for 1 minute.

The final mixture was compressed 70 mg on 7/32 FFBE punches at 4-6 kg hardness and about 0.2159 cm (0.085") gauge. One thousand tablets were produced from the composition.

### Examples 2-4

Using the same procedure described above, tablets were produced using the following ingredients:

| Ingredient | Quantity (grams) | | |
|---|---|---|---|
| | Ex. 2 | Ex. 3 | Ex. 4 |
| Ethinyl oestradiol, USP (0.5% dilution, w/5% excess) | 2.00 | 1.00 | 2.00 |
| Norethindrone acetate, USP | 0.50 | 1.00 | 1.00 |

| Lactose, Hydrous Fast Flo, USP | | | |
|---|---|---|---|
| Initial Quantity: | 8.46 | 8.56 | 8.36 |
| Added Quantity: | 33.84 | 34.24 | 33.44 |
| Corn Starch, NF | 7.00 | 7.00 | 7.00 |
| Microcrystalline cellulose | 17.50 | 17.50 | 17.50 |
| Calcium stearate | 0.70 | 0.70 | 0.70 |

## Claims

1. The use of a composition comprising 0.005 to 0.050 mg of ethinyl oestradiol and 0.1 to 1.0 mg norethindrone acetate for use in the manufacture of a medicament for the treatment of osteoporosis by the daily administration of the composition for 20 to 30 days of a 30 to 35 day cycle.

2. Use according to claim 1, wherein the treatment comprises administration of the composition for approximately 28 days of an approximately 30 day cycle.

3. Use according to any preceding claim, wherein there is present
(a) 0.001 to 0.1 parts by weight of the ethinyl oestradiol and
(b) 0.1 to 2.0 parts by weight of the norethindrone acetate.

4. Use according to any preceding claim, wherein there is present
(a) 0.005 to 0.05 parts by weight of the ethinyl oestradiol and
(b) 0.1 to 1.0 parts by weight of the norethindrone acetate.

5. A package for use in the treatment of osteoporosis by daily administration of a medicament for 20 to 30 days of a cycle lasting 30 to 35 days, which package contains a medicament which includes as individual daily doses a composition comprising 0.005 to 0.050 mg of ethinyl oestradiol and 0.1 to 1.0 mg of norethindrone acetate, the medicament being arranged in the form of a dosage unit containing a sufficient quantity of daily doses for daily administration for 20 to 30 days of a cycle lasting 30 to 35 days.

6. A package according to claim 5, wherein the medicament is arranged in a dosage unit containing 28 daily doses for daily adminstration for 28 days of a 30 day cycle.

7. A package according to claim 5 or 6, wherein the package is for use according to any one of claims 1 to 4.

## Patentansprüche

1. Verwendung einer Zusammensetzung, welche 0,005 bis 0,050 mg Ethinylöstradiol und 0,1 bis 1,0 mg Norethisteronacetat umfaßt, für einen Einsatz bei der Herstellung eines Medikamentes zur Behandlung von Osteoporose durch tägliche Verabreichung der Zusammensetzung während 20 bis 30 Tagen eines Zyklus von 30 bis 35 Tagen.

2. Verwendung nach Anspruch 1, wobei die Behandlung die Verabreichung der Zusammensetzung während ungefähr 28 Tagen eines Zyklus von ungefähr 30 Tagen umfaßt.

3. Verwendung nach einem der vorhergehenden Ansprüche, wobei
(a) 0,001 bis 0,1 Gewichtsteile des Ethinylöstradiols und
(b) 0,1 bis 2,0 Gewichtsteile des Norethisteronacetats vorliegen.

4. Verwendung nach einem der vorhergehenden Ansprüche, wobei
(a) 0,005 bis 0,05 Gewichtsteile des Ethinylöstradiols und
(b) 0,1 bis 1,0 Gewichtsteile des Norethisteronacetats vorliegen.

5. Packung, zur Verwendung bei der Behandlung von Osteoporose durch tägliche Verabreichung eines Medikamentes während 20 bis 30 Tagen eines 30 bis 35 Tage dauernden Zyklus, welche Packung ein Medikament enthält, das als tägliche Einzeldosen eine Zusammensetzung enthält, die 0,005 bis 0,050 mg Ethinylöstradiol und 0,1 bis 1,0 mg Norethisteronacetat umfaßt, wobei das Medikament in Form einer Dosierungseinheit vorgesehen ist, die eine ausreichenden Menge von Tagesdosen zur täglichen Verabreichung während 20 bis 30 Tagen eines 30 bis 35 Tage dauernden, Zyklus enthält.

6. Packung nach Anspruch 5, bei welcher das Medikament in einer Dosierungseinheit vorgesehen ist, die 28 Tagesdosen zur täglichen Verabreichung während 28 Tagen eines Zyklus von 30 Tagen enthält.

7. Packung nach Anspruch 5 oder 6, wobei die Packung zur Verwendung nach einem der Ansprüche 1 bis 4 bestimmt ist.

## Revendications

1. Utilisation d'une composition comprenant de 0,005 à 0,050 mg d'éthinyloestradiol et de 0,1 à 1,0 mg d'acétate de noréthindrone pour la fabrication d'un médicament pour le traitement de l'ostéoporose par administration quotidienne de la composition pendant 20 à 30 jours d'un cycle de 30 à 35 jours.

2. Utilisation selon la revendication 1, dans laquelle le traitement comprend l'administration de la composition pendant approximativement 28 jours d'un cycle d'approximativement 30 jours.

3. Utilisation selon l'une quelconque des revendications précédentes, dans laquelle sont présents:
- (a) 0,001 à 0,1 partie en poids d'éthinyloestradiol, et
- (b) 0,1 à 2,0 parties en poids d'acétate de noréthindrone.

4. Utilisation selon l'une quelconque des revendications précédentes, dans laquelle sont présents:
- (a) 0,005 à 0,05 partie en poids d'éthinyloestradiol, et
- (b) 0,1 à 1,0 partie en poids d'acétate de noréthindrone.

5. Un emballage contenant, pour être employé dans le traitement de l'ostéoporose par administration quotidienne d'un médicament pendant 20 à 30 jours d'un cycle de 30 à 35 jours, un médicament qui contient des doses quotidiennes individuelles, d'une composition comprenant de 0,005 à 0,050 mg d'éthinyloestradiol et de 0,1 à 1,0 mg d'acétate de noréthindrone, le médicament étant sous la forme d'une unité de dosage contenant une quantité suffisante de doses quotidiennes pour une administration quotidienne pendant 20 à 30 jours d'un cycle de 30 à 35 jours.

6. Un emballage selon la revendication 5, dans lequel le médicament est sous la forme d'une unité de dosage contenant 28 doses quotidiennes pour une administration quotidienne pendant 28 jours d'un cycle de 30 jours.

7. Un emballage selon la revendication 5 ou 6, dans lequel l'emballage est destiné à une utilisation selon l'une quelconque des revendications 1 à 4.
